# EUROPÄISCHE PATENTANMELDUNG

(11) **EP 4 385 613 A1**
(43) Veröffentlichungstag der Anmeldung: **19.06.2024**
(21) Anmeldenummer: 22213010.6
(22) Anmeldetag: 13.12.2022
(51) Int. Cl.: B01F 31/55, B01F 33/501, B01F 35/71, B01F 101/20, A61B 17/88

(54) **VORRICHTUNG ZUM MISCHEN VON KNOCHENZEMENT**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Eine Vorrichtung (10) zum Mischen von Knochenzement (34) umfasst einen ersten Hohlkörper (11), der einen ersten Hohlraum (12) zur Aufnahme eines Pulvers (14) umschließt und eine erste Öffnung (15) aufweist, und einen zweiten Hohlkörper (21), der einen zweiten Hohlraum (22) zur Aufnahme einer Ampulle (24) umschließt und eine zweite Öffnung (25) aufweist. Der erste Hohlkörper (11) und der zweite Hohlkörper (21) sind derart miteinander verbindbar, dass die erste Öffnung (15) und die zweite Öffnung (25) strömungstechnisch miteinander verbunden sind. Der erste Hohlkörper (11) ist elastisch, so dass ein Inhalt des ersten Hohlkörpers (11) durch Kneten von außen gemischt werden kann.

## Beschreibung

Die Erfindung betrifft eine Vorrichtung zum Lagern und/oder Mischen von Knochenzement.

Knochenzement wird üblicherweise durch Mischen eines Pulvers mit einer Flüssigkeit hergestellt. Beispielsweise sind Polymethylmethacrylat-(PMMA)-Knochenzemente bekannt, die aus einer flüssigen Monomerkomponente und einer Pulverkomponente zusammengesetzt sind. Die Monomerkomponente enthält im Allgemeinen das Monomer Methylmethacrylat und insbesondere einen darin gelösten Aktivator wie z. B. N,N-Dimethyl-p-toluidin. Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind und insbesondere einen Röntgenopaker und/oder den Initiator Dibenzoylperoxid auf. Beim Vermischen der Pulverkomponente mit der Monomerkomponente entsteht, z. B. durch Quellung der Polymere der Pulverkomponente im Methylmethacrylat, ein plastisch verformbarer Teig, der eigentliche Knochenzement. Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates kann sich die Viskosität des Zementteigs erhöhen, bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können. Andere Nachteile sind die Notwendigkeit, die Mengen manuell abzumessen und die Nicht immer sichergestellte ausreichende Durchmischung.

Zur Vermeidung von Lufteinschlüssen im Knochenzementteig sind Vakuum-Zementiersysteme bekannt, beispielsweise US 6,033,105 A, US 5,624,184 A oder US 4,671,263 A. Hier besteht die Notwendigkeit, ein Vakuum anzulegen. Eine Weiterentwicklung in der Zementiertechnik stellen Zementiersysteme dar, in denen sowohl das Zementpulver als auch die Monomerflüssigkeit bereits in separaten Kompartimenten verpackt sind und unmittelbar vor der Zementapplikation im Zementiersystem miteinander vermischt werden. Solche geschlossenen Full-Prepacked-Mischsysteme sind unter anderen in den Dokumenten EP0380867B1, EP0796653B1, oder EP0692229B1 beschrieben.

Mischvorrichtungen, bei denen eine externe Vorrichtung zum Herauspressen des Knochenzements verwendet wird, sind in WO9416951A1, EP1741413B1, EP3054880B1 und DE19718648A1 offenbart. Mischvorrichtungen, bei denen ein Vakuum angelegt wird, um den Transport oder das Mischen einer Komponente oder des Knochenzements zu ermöglichen, sind aus DE102009031178B3, US8662736B2 und EP3093067B1 bekannt. EP2393456B1 beschreibt eine Mischvorrichtung, bei der die Flüssigkeit mittels eines Überdrucks in das Pulver gedrückt wird.

Es ist die Aufgabe der Erfindung, eine besonders einfache und kostengünstige Vorrichtung zum Mischen der Komponenten zur Herstellung von Knochenzement und zum Austragen des hergestellten Knochenzements bereitzustellen. Insbesondere ist es Aufgabe der Erfindung, die aus dem Stand der Technik bekannten Nachteile zumindest teilweise zu beheben.

Die Aufgabe wird gelöst durch die Vorrichtung zum Mischen von Knochenzement gemäß Anspruch 1. Vorteilhafte Ausgestaltungen sind in den Unteransprüchen angegeben.

Zur Lösung der Aufgabe dient eine Vorrichtung zum Mischen von Knochenzement. Diese umfasst einen ersten Hohlkörper, der einen ersten Hohlraum zur Aufnahme eines Pulvers umschließt und eine erste Öffnung aufweist. Die Vorrichtung umfasst ferner einen zweiten Hohlkörper, der einen zweiten Hohlraum zur Aufnahme einer Ampulle umschließt und eine zweite Öffnung aufweist. Der erste Hohlkörper und der zweite Hohlkörper sind derart miteinander verbindbar, dass die erste Öffnung und die zweite Öffnung strömungstechnisch miteinander verbunden sind. Der erste Hohlkörper ist elastisch, so dass ein Inhalt des ersten Hohlkörpers durch Kneten von außen gemischt werden kann.

Durch das Kneten von außen kann besonders einfach und ohne zusätzliche Geräte ein Mischen der Komponenten erfolgen. Eine Mischstange oder ähnliches entfällt, wodurch der technische Aufwand und die Fehleranfälligkeit wesentlich verringert werden. Die Vorrichtung ist sehr robust. Es werden im einfachsten Fall lediglich zwei Teile benötigt, die zudem kostengünstig hergestellt werden können. Die Handhabung ist sehr einfach und wenig fehleranfällig.

Die Elastizität des ersten Hohlkörpers ermöglicht typischerweise ein Zusammendrücken des ersten Hohlkörpers, so dass beispielsweise darin befindliches Gas, wie z. B. Luft, herausgepresst werden kann. Auf diese Weise ändert sich das Volumen des ersten Hohlraums während des Mischens und es kann eine besonders gute Durchmischung erreicht werden. Beispielsweise kann im ersten Hohlraum befindliches Gas vorübergehend oder dauerhaft in den zweiten Hohlraum gedrückt werden.

Ein Hohlkörper ist ein Körper mit einer Wandung, die zumindest einen Hohlraum umschließt. Der Hohlraum kann geöffnet oder geschlossen sein. Der erste Hohlkörper kann gummielastisch sein und/oder aus Gummi hergestellt sein. Der erste Hohlkörper kann auch als Ampullenhalter bezeichnet werden.

Die zweite Öffnung ist zumindest für Flüssigkeiten durchlässig. Insbesondere ist die zweite Öffnung für Gase und Flüssigkeiten durchlässig. Der Begriff Gas bezieht sich in der vorliegenden Anmeldung lediglich auf den Aggregatzustand und umfasst auch Gasgemische wie z. B. Luft. Die zweite Öffnung ist so dimensioniert, dass ein Austreten der Ampulle durch die zweite Öffnung unmöglich ist. Die erste Öffnung ist insbesondere so ausgestaltet, dass ein Pulver durch die Öffnung in den ersten Hohlraum eingebracht werden kann und/oder dass eine hergestellte Mischung, insbesondere ein Knochenzement, durch die Öffnung hindurch aus dem ersten Hohlraum austreten kann.

Die strömungstechnische Verbindung der ersten Öffnung mit der zweiten Öffnung meint eine derartige Verbindung, dass Flüssigkeit oder Gas unter Abschluss von der Umgebung von der ersten Öffnung zur zweiten Öffnung und insbesondere auch zurück strömen kann.

Das Kneten von außen dient typischerweise dem Mischen der flüssigen Komponente und der Pulverkomponente zur Herstellung des Knochenzements. Insbesondere ist ein manuelles Kneten gemeint. Beim Kneten können beispielsweise ein oder mehrere Bereiche des ersten Hohlkörpers abwechselnd zusammengepresst und losgelassen werden. Es kann ebenso fortschreitend Druck auf unterschiedliche Positionen des ersten Hohlkörpers ausgeübt werden. insbesondere ist der erste Hohlkörper derart elastisch, dass eine zeitweilige Verringerung des Außendurchmessers des ersten Hohlkörpers um 50%, insbesondere um 70% gegenüber seiner unbelasteten Ausdehnung möglich ist. Durch die Elastizität nimmt der erste Hohlkörper nach Wegfall der äußeren Kraft wieder zumindest im Wesentlichen seine ursprüngliche Ausdehnung ein.

Der erste Hohlkörper kann so ausgestaltet sein, dass ein manuelles Herausdrücken des hergestellten Knochenzements durch Ausübung einer manuellen Kraft von außen möglich ist. Auf diese Weise ist auf das Austragen des Knochenzements ohne ein zusätzliches Gerät zur Ausübung einer Kraft oder Erzeugung eines Drucks oder Unterdrucks möglich.

In einer Ausgestaltung weist der zweite Hohlkörper ferner eine dritte Öffnung auf, durch welche ein Gas aus dem zweiten Hohlkörper ausströmen kann. Wenn der erste Hohlkörper ein Gas enthält, kann das Gas aus dem ersten Hohlkörper durch den zweiten Hohlkörper und die dritte Öffnung hindurch und auf diese Weise aus der Vorrichtung herausgedrückt werden. Dies kann typischerweise durch Ausüben einer manuellen Druckkraft auf den ersten Hohlkörper erfolgen. Bei Wegfall der äußeren Kraft kann ein Unterdruck im ersten Hohlkörper erzeugt werden, der die insbesondere zwischenzeitlich aus der Ampulle ausgetretene Flüssigkeit aus dem zweiten Hohlraum in den ersten Hohlraum hinein saugt. Im Vergleich zu einem Drücken der Flüssigkeit wird bei einem Saugen zum Zielort sichergestellt, dass die Flüssigkeit ausschließlich am gewünschten Zielort ankommt. Selbst im Falle eines Defekts kann die Flüssigkeit so nicht an einen anderen Ort gedrückt werden.

Es ist zum Ansaugen nicht notwendig, ein externes Vakuum anzulegen. So kann der gerätetechnische Aufwand bei der Verwendung minimiert werden und die Anwendung ist so unabhängig vom Vorhandensein einer externen Vakuumpumpe. Es ist kein Pumpkolben notwendig, um die Monomerflüssigkeit zum Zementpulver zu fördern.

Die dritte Öffnung ist insbesondere an einer der zweiten Öffnung abgewandten Seite, bevorzugt an einer der zweiten Öffnung gegenüberliegenden Seite angeordnet. So kann die Vorrichtung beim Herausdrücken des Gases so gehalten werden, dass die dritte Öffnung nach oben zeigt. Das im ersten Hohlraum befindliche Gas kann somit vollständig herausgedrückt werden. Ein maximaler Unterdruck kann so erzeugt werden und eine maximale Menge der Flüssigkeit kann in den ersten Hohlraum strömen.

Die dritte Öffnung kann beispielsweise mit einem gasdurchlässigen Verschluss, beispielsweise einer gasdurchlässigen Kappe, verschlossen sein. Auf diese Weise wird ein Eindringen von Verschmutzungen verhindert. Die dritte Öffnung ist insbesondere so dimensioniert, dass ein Austreten der Ampulle durch die dritte Öffnung unmöglich ist

Insbesondere ist die dritte Öffnung so eingerichtet, dass ein Gasströmung in beide Richtungen möglich ist. Eine Sterilisation mit Gas wie z. B. Ethylendioxid ist auf diese Weise einfach möglich. Insbesondere kann die Sterilisation im verbundenen Zustand der beiden Hohlkörper erfolgen. In diesem Fall kann das Gas auch an und in das Pulver gelangen und dieses sterilisieren.

Bevorzugt ist der erste Hohlkörper dazu eingerichtet, nach einer Verformung eine Rückstellkraft auszuüben, die der Verformung entgegenwirkt. Auf diese Weise wird ein Kneten zum Durchmischen im Vergleich zu etwa einem flexiblem Beutel erleichtert, da der erste Hohlkörper lediglich zusammengedrückt werden muss und die nach außen gerichtete Bewegung durch die Rückstellkraft automatisch erfolgt.

Bevorzugt ist für eine elastische Verformung des ersten Hohlkörpers eine geringere Kraft notwendig als für eine elastische Verformung des zweiten Hohlkörpers, sofern eine solche möglich ist. Auf diese Weise wird sichergestellt, dass im verbundenen Zustand beider Hohlkörper Flüssigkeit aus dem zweiten Hohlkörper in den ersten Hohlkörper gesaugt wird, wenn durch ein Zusammenpressen des ersten Hohlkörpers ein Unterdruck entsteht. Es wird verhindert, dass sich der zweite Hohlkörper verformt, was das Ansaugen der Flüssigkeit in den ersten Hohlkörper verhindern würde. Beispielsweise kann dies erreicht werden durch einen geringere Wandstärke des ersten Hohlkörpers im Vergleich zum zweiten Hohlkörper und/oder durch ein weicheres oder flexibleres Material des ersten Hohlkörpers.

In einer Ausgestaltung ist der zweite Hohlkörper zumindest bereichsweise derart elastisch, dass der zweite Hohlkörper von außen geknickt werden kann, um eine im zweiten Hohlraum befindliche Ampulle aufzubrechen.

Insbesondere erfolgt das Aufbrechen an einer Sollbruchstelle der zweiten Ampulle. Derartige Ampullen sind üblicherweise aus Glas hergestellt und/oder haben einen Ampullenkörper, der einstückig mit einem Kopf verbunden ist. Der Kopf kann durch Brechen vom Körper gelöst werden, sodass die in der Ampulle enthaltene Substanz, insbesondere Flüssigkeit, freigesetzt wird. Insbesondere ist ein manuelles Knicken gemeint. Bevorzugt ist am zweiten Hohlkörper im Bereich der Sollbruchstelle ein Formelement vorhanden, an dem die Position der Sollbruchstelle von außen erkennbar ist.

Insbesondere ist eine Wandung des zweiten Hohlkörpers im Bereich der Sollbruchstelle im Vergleich zu anderen Stellen der Wandung des zweiten Hohlkörpers besonders biegsam. Dies kann z. B. auf einfache Weise durch eine lokal verringerte Wandstärke erreicht werden. Mit anderen Worten weist der zweite Hohlkörper in diesem Fall eine Sollknickstelle auf, bei der sich bei Ausübung einer insbesondere manuellen Kraft von außen ein Knick einstellen wird. So kann ein besonders einfaches und wenig fehleranfälliges Öffnen der Ampulle ermöglicht werden.

In einer Ausführungsform ist die Vorrichtung so ausgestaltet, dass der Kopf der Ampulle in Richtung der zweiten Öffnung weist. So muss die Flüssigkeit nicht am Körper der Ampulle vorbei strömen, um aus der zweiten Öffnung herauszufließen.

In einer Ausgestaltung weist der zweite Hohlkörper ein Filterelement auf. Das Filterelement ist so angeordnet, dass eine durch die zweite Öffnung strömende Strömung gefiltert wird.

Ein Filterelement ist ein Element, das ein Filtern eines Mediums ermöglicht, um Partikel aus dem Medium zu entfernen. Das Filterelement kann beispielsweise als Sieb oder als Schüttung eines insbesondere porösen Materials ausgestaltet sein.

Die Strömung durch die zweite Öffnung kann z. B. eine Gasströmung, eine Strömung einer Flüssigkeit, eine Strömung eines Feststoffes, beispielsweise eines Pulvers, oder eine beliebige Mischung aus den Genannten sein. Das Filterelement kann beispielsweise verhindern, dass Pulver in den zweiten Hohlraum gelangt, dass Glassplitter aus dem zweiten Hohlraum heraus gelangen und/oder dass hergestellter Knochenzement in den zweiten Hohlraum gelangt.

Insbesondere ist das Filterelement im Bereich des äußeren Endes des zweiten Hohlkörpers angeordnet. Beispielsweise verschließt das Filterelement die zweite Öffnung im Wesentlichen bündig. Einige wenige Millimeter Abweichung können dabei toleriert werden. So kann ein Eindringen von Substanzen in den zweiten Hohlkörper vollständig verhindert werden. Zudem ist das Filterelement besonders einfach montierbar.

In einer Ausgestaltung umfasst die Vorrichtung eine im zweiten Hohlraum aufgenommene oder aufnehmbare Ampulle. Typischerweise entspricht das Volumen des ersten Hohlraums im Wesentlichen dem Volumen der Ampulle. Mit anderen Worten ist der Hohlraum, wenn die Ampulle im Hohlraum angeordnet ist, zumindest im Wesentlichen vollständig ausgefüllt. Typischerweise entspricht eine Innenkontur des zweiten Hohlraums zumindest im Wesentlichen einer Außenkontur der Ampulle. Insbesondere enthält die Ampulle eine Flüssigkeit, beispielsweise zur Herstellung von Knochenzement. Die Ampulle kann z. B. ein Monomer enthalten.

In einer Ausgestaltung umfasst die Vorrichtung das im ersten Hohlraum aufgenommene oder aufnehmbare Pulver. Insbesondere handelt es sich um Pulver zur Herstellung von Knochenzement, bevorzugt um Polymethylmethacrylat-Knochenzementpulver. Insbesondere ist oder wird der ersten Hohlkörper nur teilweise mit dem Pulver gefüllt. Dies erleichtert das Mischen, da eine bessere Beweglichkeit der zu mischenden Komponenten sowie der Mischung erreicht wird. Beispielsweise wird ein volumetrischer Anteil von mehr als 30%, bevorzugt mehr als 40% und in einer Ausführungsform ungefähr 50% oder mehr und/oder von weniger als 75%, bevorzugt weniger als 60% des ersten Hohlraums mit dem Pulver gefüllt. Insbesondere ist das verbleibende Volumen des ersten Hohlraums mit einem Gas gefüllt.

Wenn die Vorrichtung sowohl die Ampulle mit der Flüssigkeit als auch das Pulver enthält, mit anderen Worten also einsatzbereit befüllt ist, kann die Vorrichtung auch als Full-Prepacked-Mischsystem bezeichnet werden. Sie ist demnach unmittelbar einsatzbereit zur Herstellung von Knochenzement. Die Vorrichtung dient insbesondere zum Lagern der beiden Komponenten sowie zum Vermischen und Austragen von Knochenzement, insbesondere Polymethylmethacrylat-Knochenzement.

In einer Ausgestaltung weist der erste Hohlraum ein Volumen auf, welches mindestens der Summe aus einem Volumen des Pulvers und einem Volumen einer in der Ampulle aufgenommenen oder aufnehmbaren Flüssigkeit entspricht. Auf diese Weise steht ein ausreichendes Volumen zur Verfügung, um eine gute Bewegung der im ersten Hohlraum befindlichen Mischung und damit eine gute Durchmischung der Komponenten zu erreichen. So kann eine gute Qualität des Knochenzements erreicht werden.

Bevorzugt entspricht das Volumen des ersten Hohlraums mindestens der Summe aus dem Volumen des Pulvers und dem doppelten Volumen der in der Ampulle aufnehmbaren Flüssigkeit. Auf diese Weise wird das Mischen weiter vereinfacht und zugleich kann eine besonders hohe Qualität des Knochenzements gewährleistet werden. In einer Ausführungsform entspricht das Volumen des ersten Hohlraums mindestens der Summe aus dem Volumen des Pulvers und dem Volumen des zweiten Hohlraums.

In einer Ausgestaltung weist der zweite Hohlraum einen Körperbereich zur Aufnahme eines Körpers der Ampulle und/oder einen Kopfbereich zur Aufnahme eines Kopfes einer Ampulle auf. Insbesondere entspricht eine Innenkontur des Körperbereichs zumindest im Wesentlichen einer Außenkontur des Körpers der Ampulle. Die Innenkontur des Körperbereichs kann z. B. einen kreiszylinderförmigen Querschnitt aufweisen. Insbesondere entspricht eine Innenkontur des Kopfbereichs zumindest im Wesentlichen einer Außenkontur des Kopfs der Ampulle. Zwischen dem Körperbereich und dem Kopfbereich kann ein Übergangsbereich vorhanden sein, in dem Kanten und/oder Übergänge gerundet ausgeführt sein können. Körperbereich und Kopfbereich sind miteinander verbunden, sodass die geschlossene, gefüllte Ampulle im zweiten Hohlraum aufgenommen werden kann.

In einer Ausgestaltung besteht zumindest eine erste Strömungsverbindung zwischen einem zum Kopfbereich weisenden Ende des Körperbereichs und der zweiten Öffnung. Auf diese Weise kann Flüssigkeit aus dem Körper der Ampulle selbst dann aus dem zweiten Hohlkörper hinaus fließen und/oder gesaugt werden, wenn der Kopfbereich und eine möglicherweise dort vorhandene zweite Strömungsverbindung blockiert oder verstopft ist, beispielsweise durch den abgebrochenen Kopf der Ampulle und/oder durch Glassplitter. Typischerweise ist die Menge des Pulvers derart bemessen, dass lediglich die in der bestimmungsgemäßen Benutzungsposition oberhalb des Anschlusses der zweiten Strömungsverbindung befindliche Menge der Flüssigkeit für die Herstellung des Knochenzements benötigt wird.

In einer Ausgestaltung besteht eine zweite Strömungsverbindung zwischen einem vom Körperbereich weg weisenden Ende des Kopfbereichs und der zweiten Öffnung. Auf diese Weise kann Flüssigkeit aus der Ampulle aus dem zweiten Hohlkörper hinaus fließen und/oder gesaugt werden. Beispielsweise verläuft ein erster Strömungskanal, der die erste Strömungsverbindung ausbildet, zwischen einem vom Körperbereich weg weisenden Ende des Kopfbereichs und der zweiten Öffnung.

Beispielsweise verläuft zumindest ein zweiter Strömungskanal, der die zweite Strömungsverbindung ausbildet, zwischen einem zum Kopfbereich weisenden Ende des Körperbereichs und dem ersten Strömungskanal. In diesem Fall dient z. B. ein unterer Bereich des Strömungskanals, der mit der zweiten Öffnung verbunden ist, als erste und zweite Strömungsverbindung. Es kann sich eine Abzweigung anschließen, in welcher der erste Strömungskanal zum Körperteil vom zweiten Strömungskanal zum Kopfteil abgezweigt wird oder umgekehrt. Mit anderen Worten können die erste Strömungsverbindung und die zweite Strömungsverbindung teilweise übereinstimmen. In einer Ausführungsform sind zwei zweite Strömungsverbindungen vorgesehen. Diese sind insbesondere gleichartig und/oder wie beschrieben als zweite Strömungskanäle ausgeführt.

In einer Ausgestaltung umschließt der zweite Hohlkörper zwei zweite Hohlräume, wobei jeder zweite Hohlraum zur Aufnahme einer Ampulle eingerichtet ist. Insbesondere ist eine gemeinsame zweite Öffnung vorgesehen, durch die Flüssigkeit aus beiden Ampullen aus dem zweiten Hohlkörper ausströmen kann. Typischerweise weist jeder zweite Hohlraum eine eigene Öffnung auf, an die sich ein jeweiliger Strömungskanal anschließt. Die Strömungskanäle können sich insbesondere im Inneren des zweiten Hohlkörpers zu einem einzigen Strömungskanal vereinigen, der mit der gemeinsamen zweiten Öffnung verbunden ist. Alternativ sind zwei insbesondere miteinander verbundene zweite Hohlkörper vorhanden, die jeweils einen Hohlraum zur Aufnahme einer Ampulle umschließen.

Die Verwendung zweier Ampullen anstelle einer größeren Ampulle ermöglicht, dass das Flüssigkeitsvolumen in der Ampulle weniger als 30 ml beträgt. Hierdurch ergeben sich Vorteile aufgrund von Einschränkungen größerer Mengen gemäß Gefahrstoffverordnungen.

In einer Ausgestaltung ist eine gemeinsame Knicklinie für beide zweiten Hohlräume vorgesehen ist, sodass durch ein einzelnes Knicken von außen beide im zweiten Hohlkörper befindliche Ampullen aufgebrochen werden können. Ein einzelnes Knicken meint eine einzelne Knickbewegung, die insbesondere mit einer einzelnen von außen aufgebrachten Kraft ausgeführt werden kann. Es ist demnach nur eine Bewegung notwendig, um beide Ampullen zu öffnen.

Insbesondere können beide Ampullen gleichzeitig aufgebrochen werden. Typischerweise sind die zweiten Hohlräume parallel zueinander angeordnet und weisen dieselbe Ausrichtung auf. Die Knicklinie verläuft dann insbesondere senkrecht zur Längsachse der zweiten Hohlräume auf der Höhe eines Übergangs zwischen einem Kopfbereich und einem Körperbereich.

In einer Ausgestaltung weist der erste Hohlkörper ein erstes Verbindungselement auf und/oder der zweite Hohlkörper weist ein zweites Verbindungselement auf. In einer Ausgestaltung sind das erste Verbindungselement und das zweite Verbindungselement so eingerichtet, dass der erste Hohlkörper und der zweite Hohlkörper manuell und lösbar miteinander verbindbar sind.

Das erste Verbindungselement und das zweite Verbindungselement werden insbesondere unmittelbar miteinander verbunden. Das erste Verbindungselement und das zweite Verbindungselement sind typischerweise als korrespondierende Elemente ausgestaltet, beispielsweise als korrespondierende Gewinde. Die Verbindung meint eine mechanische Verbindung derart, dass die beiden Hohlkörper fest aneinander gekoppelt sind und auf diese Weise beispielsweise gemeinsam bewegt werden können

Insbesondere sind die Verbindungselemente so ausgestaltet, dass im verbundenen Zustand eine Längsachse des ersten Hohlkörpers und eine Längsachse des zweiten Hohlkörpers übereinanderliegen. So kann die Flüssigkeit über einen geradlinigen, kurzen Strömungsweg in das Pulver strömen.

In einer Ausgestaltung weist die Vorrichtung ferner einen Adapter auf, welcher mit dem ersten Verbindungselement verbindbar ist. Bevorzugt ist der Adapter so eingerichtet, dass eine Mischung zur Herstellung von Knochenzement im ersten Hohlkörper zurückgehalten wird.

Mit anderen Worten wird mit verbundenem Adapter verhindert, dass die Mischung aus dem ersten Hohlkörper ausströmt. Auf diese Weise kann der Inhalt des ersten Hohlkörpers mit verbundenem Adapter gemischt werden. Dies erleichtert das Mischen, da die Vorrichtung, bei der der Adapter anstelle des zweiten Hohlkörpers am ersten Hohlkörper angeordnet ist kleiner und handlicher ist.

Insbesondere ist der Adapter so eingerichtet, dass Gas weiterhin aus dem ersten Hohlkörper austreten kann. So wird das Mischen weiter verbessert, da ein effektiveres Kneten möglich wird. Insbesondere umfasst der Adapter ein Filterelement, welches die Mischung zurückhält und/oder Gas durchlässt. Das Filterelement kann so ausgestaltet sein wie das Filterelement des zweiten Hohlkörpers.

In einer Ausgestaltung weist die Vorrichtung ferner ein Austragelement mit einem dritten Verbindungselement auf. Insbesondere kann das Austragelement an das erste Verbindungselement des ersten Hohlkörpers angeschlossen werden.

Das Austragelement dient dazu, den hergestellten Knochenzement aus dem ersten Hohlkörper auszutragen. Insbesondere kann dies durch Zusammenpressen des ersten Hohlkörpers erfolgen. Das dritte Verbindungselement wird typischerweise anstelle des zweiten Verbindungselements nach dem Lösen des zweiten Verbindungselements vom ersten Verbindungselement mit dem ersten Verbindungselement verbunden. Verbinden meint eine mechanische Kopplung derart, dass eine strömungstechnische Verbindung zwischen dem ersten Hohlraum und einem Inneren des Austragelements besteht. Insbesondere kann das Austragelement manuell mit dem ersten Verbindungselement verbunden werden. Insbesondere ist eine formschlüssige und/oder kraftschlüssige Verbindung gemeint. Auf diese Weise wird eine besonders einfache Handhabung ermöglicht.

Bevorzugt hat das Austragelement eine Düse, durch die der Knochenzement exakt an eine Zielposition befördert werden kann. Bevorzugt ist die Düse an der dem dritten Verbindungselement gegenüberliegenden Seite des Austragelements angeordnet.

In einer Ausgestaltung ist der erste Hohlkörper aus einem Material mit einer Shore A-Härte zwischen 30 und 80 hergestellt.

In einer Ausführungsform ist der erste Hohlkörper aus einem Material mit einer Shore A-Härte größer als 30, insbesondere größer oder gleich 40 und bevorzugt größer oder gleich 50 und/oder kleiner als 80, bevorzugt kleiner als 70, insbesondere kleiner als 60 hergestellt. Derartige Materialien haben sich hinsichtlich der benötigten Elastizität als besonders vorteilhaft erwiesen.

Ein weiterer Aspekt der Erfindung ist ein Verfahren zum Herstellen von Knochenzement, insbesondere unter Verwendung der erfindungsgemäßen Vorrichtung. Das Verfahren umfasst Kneten eines elastischen ersten Hohlkörpers von außen, insbesondere manuelles Kneten, zum Mischen eines Inhalts des ersten Hohlkörpers. Insbesondere umschließt der erste Hohlkörper einen ersten Hohlraum zur Aufnahme eines Pulvers und weist eine erste Öffnung auf. Insbesondere ist ein zweiter Hohlkörper vorhanden. Insbesondere umschließt der zweite Hohlkörper einen zweiten Hohlraum zur Aufnahme einer Ampulle und weist eine zweite Öffnung auf. Insbesondere sind während des Knetens die erste Öffnung und die zweite Öffnung strömungstechnisch miteinander verbunden. Insbesondere wird der erste Hohlkörper so positioniert, dass die erste Öffnung nach oben weist.

In einer Ausführungsform umfasst das Verfahren ein Ausüben einer Kraft auf den ersten Hohlkörper von außen. Auf diese Weise kann ein im ersten Hohlkörper befindliches Gas durch die erste Öffnung aus dem ersten Hohlkörper herausströmen. Insbesondere kann das Gas durch die zweite Öffnung in den zweiten Hohlkörper hineinströmen, an einer im zweiten Hohlraum befindlichen Ampulle vorbeiströmen und/oder den zweiten Hohlraum durch eine dritte Öffnung des zweiten Hohlkörpers verlassen. In einer Ausführungsform umfasst das Verfahren ein Knicken des zweiten Hohlkörpers von außen, so dass die Ampulle aufgebrochen wird und eine in der Ampulle befindliche Flüssigkeit freigesetzt wird. In einer Ausführungsform umfasst das Verfahren ein Lösen der auf den ersten Hohlkörper ausgeübten Kraft. Auf diese Weise kann durch eine Rückstellkraft einer Wandung des ersten Hohlkörpers ein Unterdruck erzeugt werden, um die freigesetzte Flüssigkeit in den ersten Hohlkörper zu saugen. Insbesondere erfolgt im Anschluss das Kneten. In einer Ausführungsform umfasst das Verfahren ein Entfernen des zweiten Hohlkörpers vom ersten Hohlkörper, ein Verbinden eines Austragelements mit dem ersten Hohlkörper und/oder ein Austragen des hergestellten Knochenzements durch Ausüben einer äußeren Kraft auf den ersten Hohlkörper.

In einer Ausführungsform wird die Kraft, mit der das Gas aus dem ersten Hohlkörper herausgepresst wird, ausgeübt, bevor die Ampulle aufgebrochen wird. In einer alternativen Ausführungsform wird zunächst die Ampulle aufgebrochen und anschließend erfolgt ein einfaches oder mehrfaches Ausüben einer Kraft auf den ersten Hohlkörper von außen. Auf diese Weise kann das Gas in einem oder mehreren Teilen ausströmen und jeweils durch den entstehenden Unterdruck eine entsprechende Portion der Flüssigkeit eingesaugt werden.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: eine perspektivische Ansicht einer erfindungsgemäßen Vorrichtung,
- Figur 2:: eine Schnittdarstellung einer erfindungsgemäßen Vorrichtung,
- Figur 3:: eine weitere Schnittdarstellung einer erfindungsgemäßen Vorrichtung,
- Figur 4:: eine weitere Schnittdarstellung einer erfindungsgemäßen Vorrichtung,
- Figur 5:: eine weitere Schnittdarstellung einer erfindungsgemäßen Vorrichtung,
- Figur 6:: eine weitere Schnittdarstellung einer erfindungsgemäßen Vorrichtung,
- Figur 7:: eine geschnittene perspektivische Ansicht einer erfindungsgemäßen Vorrichtung,
- Figur 8:: eine perspektivische Ansicht einer erfindungsgemäßen Vorrichtung,
- Figur 9:: eine perspektivische Ansicht einer erfindungsgemäßen Vorrichtung, sowie
- Figur 10:: eine geschnittene Detailzeichnung einer erfindungsgemäßen Vorrichtung.

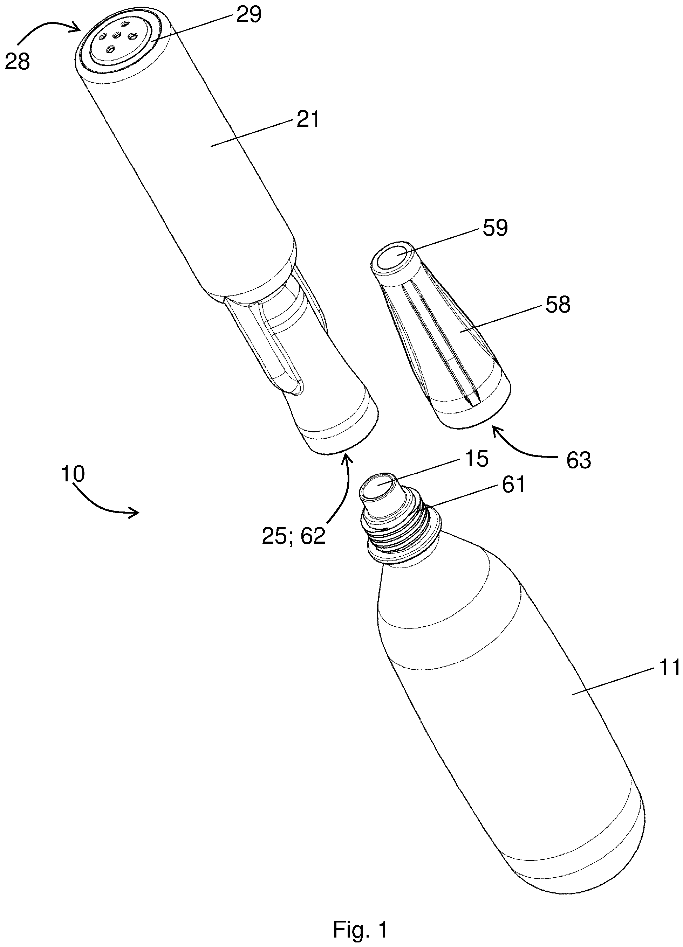

Figur 1 zeigt eine Vorrichtung 10 zum Mischen von Knochenzement. Die Vorrichtung 10 umfasst einen ersten Hohlkörper 11, einen zweiten Hohlkörper 21 und ein optionales Austragelement 58. Insbesondere ist jeder der Hohlkörper 11, 21 aus Gummi und/oder Kunststoff hergestellt. Der erste Hohlkörper 11 umfasst eine erste Öffnung 15. Im Bereich der ersten Öffnung 15 ist ein erstes Verbindungselement 61 in Form eines Außengewindes angeordnet. Der zweite Hohlkörper 21 umfasst eine zweite Öffnung 25 sowie eine dritte Öffnung 28. Die dritte Öffnung 28 ist mit einem gasdurchlässigen Deckel 29 verschlossen. Der Deckel 21 kann abnehmbar ausgestaltet sein, um ein Einstecken einer Ampulle in den zweiten Hohlkörper 21 zu ermöglichen. Beide Öffnungen 25, 28 sind insbesondere zentral an gegenüberliegenden Seiten des zweiten Hohlkörpers 21 angeordnet.

Im Bereich der zweiten Öffnung 25 befindet sich ein zweites Verbindungselement 62 in Form eines Innengewindes. Der erste Hohlkörper 11 und der zweite Hohlkörper 21 kann mittels der Verbindungselemente 61, 62 mechanisch miteinander verbunden werden. Im verbundenen Zustand der beiden Hohlkörper 11, 21 sind die erste Öffnung 15 und die zweite Öffnung 25 strömungstechnisch miteinander verbunden.

Das Austragelement 58 umfasst eine Düse 59 zum Austragen von Knochenzement sowie ein drittes Verbindungselement 63 in Form eines Innengewindes, welches insbesondere dem Innengewinde des zweiten Verbindungselements 62 entspricht. Auf diese Weise kann das Austragelement 58 anstelle des zweiten Hohlkörpers 21 mit dem ersten Verbindungselement 61 des ersten Hohlkörpers 11 mechanisch verbunden werden.

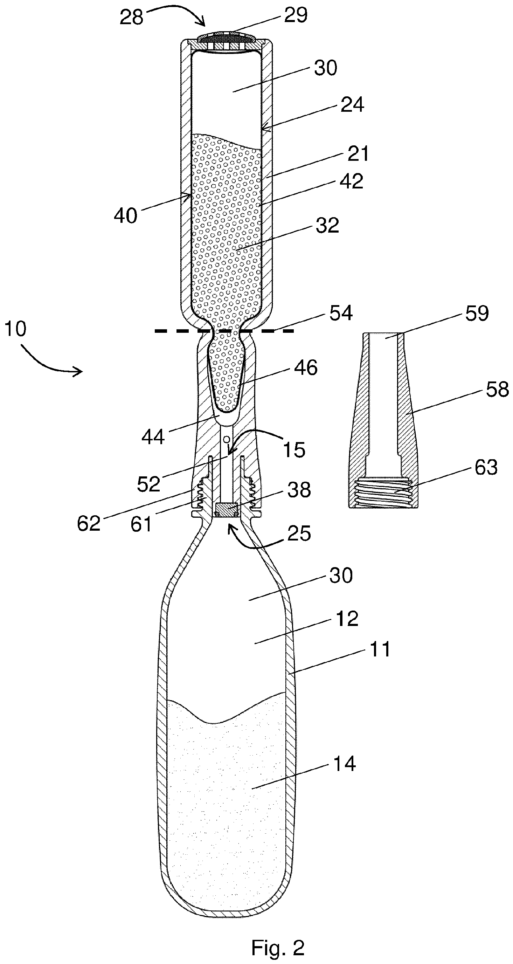

Figur 2 zeigt die Vorrichtung 10, die der Vorrichtung aus Figur 1 entsprechen kann, in einer geschnittenen Ansicht. Der erste Hohlkörper 11 und der zweite Hohlkörper 21 sind wie oben beschrieben mittels der Verbindungselemente 61, 62 mechanisch miteinander verbunden. Der erste Hohlkörper 11 umschließt einen ersten Hohlraum 12, welcher teilweise mit Pulver 14 und teilweise mit einem Gas 30, beispielsweise Luft, gefüllt ist. Der zweite Hohlkörper 21 umschließt einen zweiten Hohlraum 22, in welchem sich eine Ampulle 24 befindet. Die Ampulle 24 ist teilweise mit einer Flüssigkeit 32 und teilweise mit einem Gas 30 gefüllt oder vollständig mit der Flüssigkeit 32 gefüllt.

Die Ampulle 24 ist aus einem im Wesentlichen kreiszylinderförmigen Körper 42 und einem daran anschließenden Kopf 46 zusammengesetzt. In Figur 2 weist der Kopf 46 nach unten.

Am Übergang zwischen dem Körper 42 und dem Kopf 46 befindet sich eine Sollbruchstelle, um den Kopf 46 vom Körper zu trennen und auf diese Weise die Flüssigkeit 32 freizusetzen. Der zweite Hohlraum 22 des zweiten Hohlkörpers 21 ist entsprechend in einen im Wesentlichen kreiszylinderförmigen Körperbereich 40 zur Aufnahme des Körpers 42 und einen daran anschließenden Kopfbereich 44 zur Aufnahme des Kopfes 46 unterteilt. Zumindest im Bereich der Sollbruchstelle ist der zweite Hohlkörper 21 elastisch, sodass die Ampulle 24 wie beschrieben durch ein Knicken von außen entlang der Knicklinie 54 geöffnet werden kann.

Eine zweite Strömungsverbindung 52 in Form eines geradlinigen Strömungskanals befindet sich zwischen dem unteren, vom Körperbereich 40 weg weisenden Ende des Kopfbereichs 44 und der zweiten Öffnung 25. Am unteren Ende des Strömungskanals im Bereich der zweiten Öffnung 25 befindet sich ein Filterelement 38, mit welchem eine durch die zweite Öffnung 25 strömende Strömung gefiltert werden kann. Auf diese Weise können z. B. Glasscherben, die beim Öffnen der Ampulle 24 entstehen, zurückgehalten werden, wenn die Flüssigkeit 32 durch die zweite Öffnung 25 strömt.

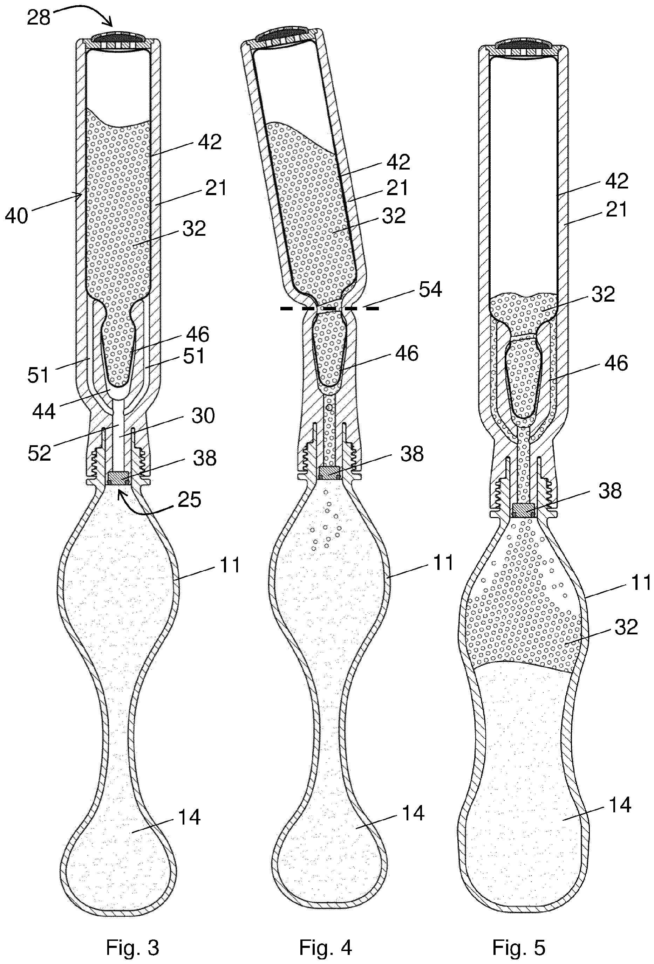

In den Figuren 3 bis 5 sind aufeinanderfolgende Schritte eines Verfahrens zur Herstellung eines Pulverzements dargestellt. Die dazu verwendete Vorrichtung 10 entspricht insbesondere der Vorrichtung aus Figur 2. In den Figuren 3 und 5 ist die Vorrichtung 10 entlang ihrer Längsachse um 90° gedreht dargestellt. Hierbei zeigt sich, dass zusätzlich zur zweiten Strömungsverbindung 52 auch zwei erste Strömungsverbindungen 51 zwischen dem zweiten Hohlraum 22 und der zweiten Öffnung 25 bestehen. Zwei beispielsweise gebogene Strömungskanäle verlaufen zwischen dem zum Kopfbereich 44 weisenden unteren Ende des Körperbereichs 40 und dem geradlinigen Strömungskanal der zweiten Strömungsverbindung 52. Die ersten Strömungsverbindungen 51 befinden sich also oberhalb oder zumindest auf derselben Höhe wie die Sollbruchstelle. Auf diese Weise kann die Flüssigkeit 32 auch dann durch die zweite Öffnung 25 strömen, wenn der Kopfbereich 44 mit dem abgebrochenen Kopf 46 der Ampulle 24 oder mit Glasscherben verstopft ist.

In Figur 3 ist dargestellt, dass eine manuelle Kraft von außen auf den ersten Hohlkörper 11 aufgebracht wird. Der erste Hohlkörper 11 wird zusammengedrückt. Oberhalb des Pulvers 14 befindliches Gas strömt auf diese Weise durch die zweite Öffnung 25 und die Strömungsverbindungen 51, 52 in den zweiten Hohlraum 22, an der Ampulle 24 vorbei und durch die dritte Öffnung 28 aus dem zweiten Hohlkörper 21 heraus nach außen. Das Filterelement 38 kann dabei verhindern, dass Pulver 14 in den zweiten Hohlkörper 21 gelangt.

Figur 4 zeigt das Knicken des zweiten Hohlkörpers 21 entlang der Knicklinie 54 durch Aufbringen manueller Kraft von außen. In der Folge wird der Kopf 46 der Ampulle 24 vom Körper 42 abgetrennt und die Flüssigkeit 32 wird freigesetzt. Die Flüssigkeit 32 beginnt, nach unten zu strömen. Nun wird die auf den ersten Hohlkörper 11 ausgeübte manuelle Kraft weggenommen. Aufgrund der durch die Wandung des ersten Hohlkörpers 11 ausgeübten Rückstellkraft bildet sich im ersten Hohlkörper 11 ein Unterdruck, der die Flüssigkeit 32 zusätzlich zur Wirkung der Schwerkraft nach unten saugt. Figur 5 zeigt den Zustand, nachdem bereits ein Großteil der Flüssigkeit 32 in den ersten Hohlkörper 11 geströmt ist. Sobald der erste Hohlkörper 11 seine ursprüngliche Form wieder angenommen hat und/oder die benötigte Menge an Flüssigkeit 32 in den ersten Hohlkörper 11 geströmt ist, kann der elastische erste Hohlkörper 11 von außen geknetet werden, um auf diese Weise das Pulver 14 mit der Flüssigkeit 32 möglichst homogen zu vermischen. Dabei verhindert das Filterelement 38, dass die Mischung in den zweiten Hohlkörper 21 strömt. Auf diese Weise wird im ersten Hohlraum 12 ein verarbeiteter Knochenzement hergestellt.

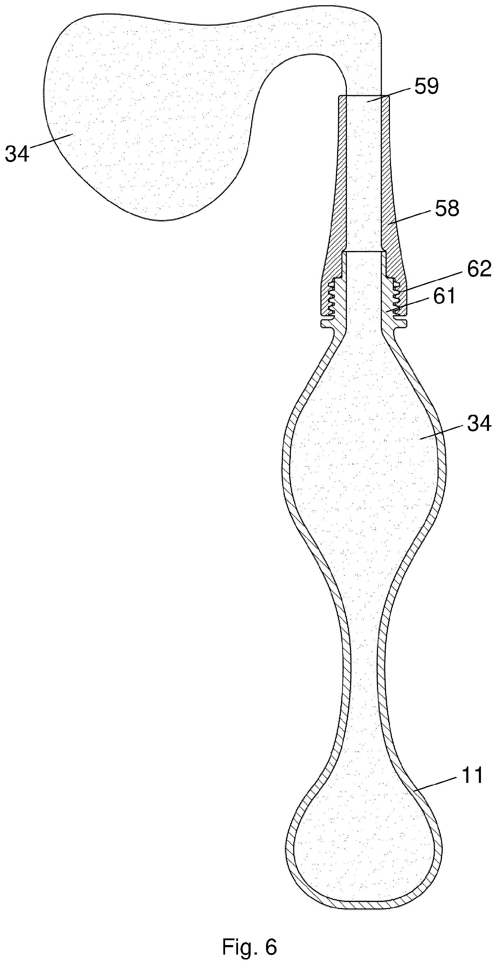

Figur 6 zeigt das Austragen des hergestellten Knochenzements. Anstelle des zweiten Hohlkörpers ist nun das Austragelement 58 am ersten Verbindungselement 61 des ersten Hohlkörpers 11 befestigt. Durch Aufbringen einer manuellen Druckkraft von außen auf den flexiblen ersten Hohlkörper 11 wird der hergestellte Knochenzement 34 durch die Düse 59 herausgedrückt.

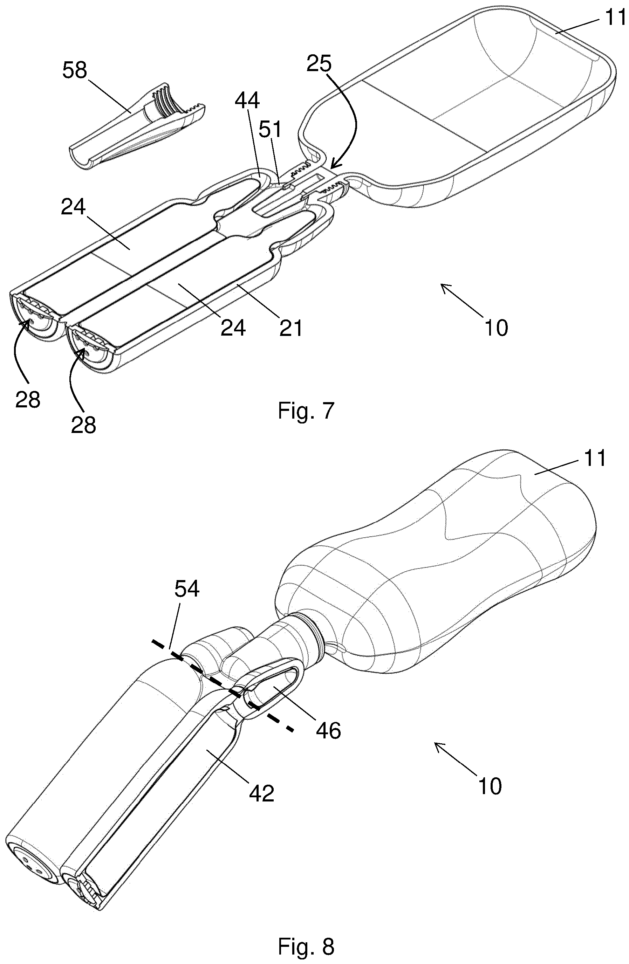

Die Figuren 7 und 8 zeigen eine andere Ausführungsform der erfindungsgemäßen Vorrichtung 10. Abweichend von den bisher beschriebenen Vorrichtungen umschließt der zweite Hohlkörper 21 hier zwei zweite Hohlräume 22. In jedem zweiten Hohlraum 22 befindet sich jeweils eine Ampulle 24. Jeder der zweiten Hohlräume 22 ist mittels einer oder mehrerer Strömungsverbindungen mit der zweiten Öffnung 25 verbunden. In Figur 8 ist dargestellt, dass eine gemeinsame Knicklinie 54 für die beiden zweiten Hohlräume 22 vorgesehen ist. So kann mit einer einzigen Knickbewegung von außen die Flüssigkeit aus beiden Ampullen 24 freigesetzt werden.

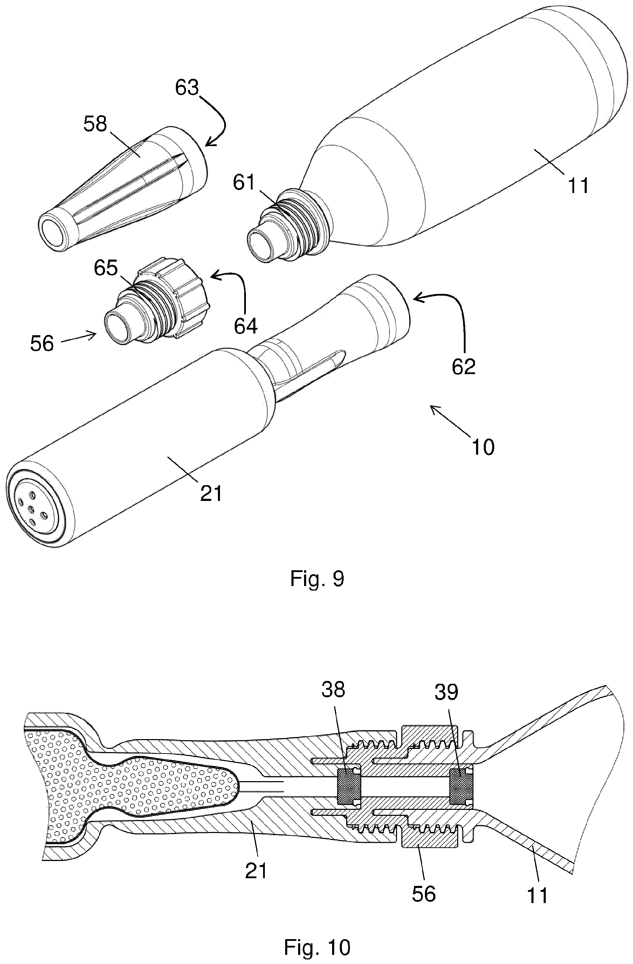

Die Figuren 9 und 10 zeigen eine andere Ausführungsform der erfindungsgemäßen Vorrichtung 10. Zusätzlich zum bereits beschriebenen umfasst die hier gezeigte Vorrichtung 10 einen Adapter 56. Dieser umfasst ein viertes Verbindungselement 64 in Form eines Innengewindes, welches im Wesentlichen dem zweiten Verbindungselement 62 entspricht. Der Adapter 56 umfasst ferner ein fünftes Verbindungselement 65 in Form eines Außengewindes, welches im Wesentlichen dem ersten Verbindungselement 61 entspricht. Somit kann der Adapter 56 auf das erste Verbindungselement 61 und auf das zweite Verbindungselement 62 aufgeschraubt werden und dem entsprechend zwischen dem ersten Hohlkörper 11 und dem zweiten Hohlkörper 21 angeordnet werden, wie in Figur 10 dargestellt ist. Die Flüssigkeit aus der Ampulle kann somit durch den Adapter 56 in den ersten Hohlraum 12 strömen.

Der Adapter 56 umfasst ferner ein zweites Filterelement 39. Dieses ist insbesondere wie das erste Filterelement 38 ausgestaltet. Das zweite Filterelement 39 befindet sich insbesondere an dem Ende des durch den Adapter 56 verlaufenen Strömungskanals, welcher bei bestimmungsgemäßem Gebrauch dem ersten Hohlkörper 11 zugewandt ist. Auf diese Weise verhindert das zweite Filterelement 39, dass die Mischung zur Herstellung von Knochenzement beim Kneten des ersten Hohlkörpers 11 aus diesem herausgelangt. Es ist also möglich, das Kneten von außen zum Mischen des Knochenzements durchzuführen, während der zweite Hohlkörper 21 vom ersten Hohlkörper 11 getrennt ist und lediglich der Adapter 56 am ersten Hohlkörper 11 angeordnet ist. Auf diese Weise ist die Vorrichtung handlicher, was das Mischen erleichtert. Insgesamt kann die Verbindung des ersten Hohlkörpers 11 und des zweiten Hohlkörpers also direkt oder indirekt, mit einem dazwischengeschalteten Adapter, erfolgen.

**Bezugszeichenliste**

| | |
|---|---|
| Vorrichtung | 10 |
| erster Hohlkörper | 11 |
| erster Hohlraum | 12 |
| Pulver | 14 |
| Erste Öffnung | 15 |
| zweiter Hohlkörper | 21 |
| zweiter Hohlraum | 22 |
| Ampulle | 24 |
| zweite Öffnung | 25 |
| dritte Öffnung | 28 |
| Deckel | 29 |
| Gas | 30 |
| Flüssigkeit | 32 |
| Knochenzement | 34 |
| Filterelement | 38 |
| Zweites Filterelement | 39 |
| Körperbereich | 40 |
| Körper | 42 |
| Kopfbereich | 44 |
| Kopf | 46 |
| erste Strömungsverbindung | 51 |
| zweite Strömungsverbindung | 52 |
| Knicklinie | 54 |
| Adapter | 56 |
| Austragelement | 58 |
| Düse | 59 |
| erstes Verbindungselement | 61 |
| zweites Verbindungselement | 62 |
| drittes Verbindungselement | 63 |
| viertes Verbindungselement | 64 |
| fünftes Verbindungselement | 65 |

## Patentansprüche

1. Vorrichtung (10) zum Mischen von Knochenzement (34), umfassend einen ersten Hohlkörper (11), der einen ersten Hohlraum (12) zur Aufnahme eines Pulvers (14) umschließt und eine erste Öffnung (15) aufweist, und einen zweiten Hohlkörper (21), der einen zweiten Hohlraum (22) zur Aufnahme einer Ampulle (24) umschließt und eine zweite Öffnung (25) aufweist, wobei der erste Hohlkörper (11) und der zweite Hohlkörper (21) derart miteinander verbindbar sind, dass die erste Öffnung (15) und die zweite Öffnung (25) strömungstechnisch miteinander verbunden sind, wobei der erste Hohlkörper (11) elastisch ist, so dass ein Inhalt des ersten Hohlkörpers (11) durch Kneten von außen gemischt werden kann.

2. Vorrichtung (10) nach Anspruch 1, **dadurch gekennzeichnet, dass** der zweite Hohlkörper (21) ferner eine dritte Öffnung (28) aufweist, durch welche ein Gas (30) aus dem zweiten Hohlkörper (21) ausströmen kann.

3. Vorrichtung (10) nach einem der vorhergehenden Ansprüche, **dadurch gekennzeichnet, dass** der zweite Hohlkörper (21) zumindest bereichsweise derart elastisch ist, dass der zweite Hohlkörper (21) von außen geknickt werden kann, um eine im zweiten Hohlraum (22) befindliche Ampulle (24) aufzubrechen.

4. Vorrichtung (10) nach einem der vorhergehenden Ansprüche, **dadurch gekennzeichnet, dass** der zweite Hohlkörper (21) ein Filterelement (38) aufweist, wobei das Filterelement (38) so angeordnet ist, dass eine durch die zweite Öffnung (25) strömende Strömung gefiltert wird.

5. Vorrichtung (10) nach einem der vorhergehenden Ansprüche, **dadurch gekennzeichnet, dass** die Vorrichtung (10) eine im zweiten Hohlraum (22) aufgenommene Ampulle (24) umfasst.

6. Vorrichtung (10) nach einem der vorhergehenden Ansprüche, **dadurch gekennzeichnet, dass** die Vorrichtung (10) ein im ersten Hohlraum (12) aufgenommenes Pulver (14) umfasst.

7. Vorrichtung (10) nach dem vorhergehenden Anspruch, **dadurch gekennzeichnet, dass** der erste Hohlraum (12) ein Volumen aufweist, welches mindestens der Summe aus einem Volumen des Pulvers (14) und einem Volumen einer in der Ampulle (24) aufnehmbaren Flüssigkeit (32) entspricht.

8. Vorrichtung (10) nach einem der vorhergehenden Ansprüche, **dadurch gekennzeichnet, dass** der zweite Hohlraum (22) einen Körperbereich (40) zur Aufnahme eines Körpers (42) der Ampulle (24) und einen Kopfbereich (44) zur Aufnahme eines Kopfes (46) einer Ampulle (24) aufweist, wobei zumindest eine erste Strömungsverbindung (51) zwischen einem zum Kopfbereich (44) weisenden Ende des Körperbereichs (40) und der zweiten Öffnung (25) besteht.

9. Vorrichtung (10) nach dem vorhergehenden Anspruch, **dadurch gekennzeichnet, dass** eine zweite Strömungsverbindung (52) zwischen einem vom Körperbereich (40) weg weisenden Ende des Kopfbereichs (44) und der zweiten Öffnung (25) besteht.

10. Vorrichtung (10) nach einem der vorhergehenden Ansprüche, **dadurch gekennzeichnet, dass** der zweite Hohlkörper (21) zwei zweite Hohlräume (22) umschließt, wobei jeder zweite Hohlraum (22) zur Aufnahme einer Ampulle (24) eingerichtet ist.

11. Vorrichtung (10) nach dem vorhergehenden Anspruch und Anspruch 3, **dadurch gekennzeichnet, dass** eine gemeinsame Knicklinie (54) für beide zweiten Hohlräume (22) vorgesehen ist, sodass durch ein einzelnes Knicken von außen beide im zweiten Hohlkörper (21) befindliche Ampullen (24) aufgebrochen werden können.

12. Vorrichtung (10) nach einem der vorhergehenden Ansprüche, **dadurch gekennzeichnet, dass** der erste Hohlkörper (11) ein erstes Verbindungselement (61) aufweist und der zweite Hohlkörper (21) ein zweites Verbindungselement (62) aufweist, wobei das erste Verbindungselement (61) und das zweite Verbindungselement (62) so eingerichtet sind, dass der erste Hohlkörper (11) und der zweite Hohlkörper (21) manuell und lösbar miteinander verbindbar sind.

13. Vorrichtung (10) nach dem vorhergehenden Anspruch, **dadurch gekennzeichnet, dass** die Vorrichtung (10) ferner einen Adapter (56) aufweist, welcher mit dem ersten Verbindungselement (61) verbindbar ist und so eingerichtet ist, dass eine Mischung zur Herstellung von Knochenzement (34) im ersten Hohlkörper (11) zurückgehalten wird.

14. Vorrichtung (10) nach einem der zwei vorhergehenden Ansprüche, **dadurch gekennzeichnet, dass** die Vorrichtung (10) ferner ein Austragelement (58) mit einem dritten Verbindungselement (63) aufweist, welches an das erste Verbindungselement (61) des ersten Hohlkörpers (11) angeschlossen werden kann.

15. Vorrichtung (10) nach dem vorhergehenden Anspruch, **dadurch gekennzeichnet, dass** der erste Hohlkörper (11) aus einem Material mit einer Shore A-Härte zwischen 30 und 80 hergestellt ist.

## Geänderte Patentansprüche

### Geänderte Patentansprüche gemäss Regel 137(2) EPÜ.

1. Vorrichtung (10) zum Mischen von Knochenzement (34), umfassend einen ersten Hohlkörper (11), der einen ersten Hohlraum (12) zur Aufnahme eines Pulvers (14) umschließt und eine erste Öffnung (15) aufweist, und einen zweiten Hohlkörper (21), der einen zweiten Hohlraum (22) zur Aufnahme einer Ampulle (24) umschließt und eine zweite Öffnung (25) aufweist, wobei der erste Hohlkörper (11) und der zweite Hohlkörper (21) derart miteinander verbindbar sind, dass die erste Öffnung (15) und die zweite Öffnung (25) strömungstechnisch miteinander verbunden sind, wobei der erste Hohlkörper (11) elastisch ist, so dass ein Inhalt des ersten Hohlkörpers (11) durch Kneten von außen gemischt werden kann, **dadurch gekennzeichnet, dass** der zweite Hohlkörper (21) ein Filterelement (38) aufweist, wobei das Filterelement (38) so angeordnet ist, dass eine durch die zweite Öffnung (25) strömende Strömung gefiltert wird.

2. Vorrichtung (10) nach Anspruch 1, **dadurch gekennzeichnet, dass** der zweite Hohlkörper (21) ferner eine dritte Öffnung (28) aufweist, durch welche ein Gas (30) aus dem zweiten Hohlkörper (21) ausströmen kann.

3. Vorrichtung (10) nach einem der vorhergehenden Ansprüche, **dadurch gekennzeichnet, dass** der zweite Hohlkörper (21) zumindest bereichsweise derart elastisch ist, dass der zweite Hohlkörper (21) von außen geknickt werden kann, um eine im zweiten Hohlraum (22) befindliche Ampulle (24) aufzubrechen.

4. Vorrichtung (10) nach einem der vorhergehenden Ansprüche, **dadurch gekennzeichnet, dass** die Vorrichtung (10) eine im zweiten Hohlraum (22) aufgenommene Ampulle (24) umfasst.

5. Vorrichtung (10) nach einem der vorhergehenden Ansprüche, **dadurch gekennzeichnet, dass** die Vorrichtung (10) ein im ersten Hohlraum (12) aufgenommenes Pulver (14) umfasst.

6. Vorrichtung (10) nach dem vorhergehenden Anspruch, **dadurch gekennzeichnet, dass** der erste Hohlraum (12) ein Volumen aufweist, welches mindestens der Summe aus einem Volumen des Pulvers (14) und einem Volumen einer in der Ampulle (24) aufnehmbaren Flüssigkeit (32) entspricht.

7. Vorrichtung (10) nach einem der vorhergehenden Ansprüche, **dadurch gekennzeichnet, dass** der zweite Hohlraum (22) einen Körperbereich (40) zur Aufnahme eines Körpers (42) der Ampulle (24) und einen Kopfbereich (44) zur Aufnahme eines Kopfes (46) einer Ampulle (24) aufweist, wobei zumindest eine erste Strömungsverbindung (51) zwischen einem zum Kopfbereich (44) weisenden Ende des Körperbereichs (40) und der zweiten Öffnung (25) besteht.

8. Vorrichtung (10) nach dem vorhergehenden Anspruch, **dadurch gekennzeichnet, dass** eine zweite Strömungsverbindung (52) zwischen einem vom Körperbereich (40) weg weisenden Ende des Kopfbereichs (44) und der zweiten Öffnung (25) besteht.

9. Vorrichtung (10) nach einem der vorhergehenden Ansprüche, **dadurch gekennzeichnet, dass** der zweite Hohlkörper (21) zwei zweite Hohlräume (22) umschließt, wobei jeder zweite Hohlraum (22) zur Aufnahme einer Ampulle (24) eingerichtet ist.

10. Vorrichtung (10) nach dem vorhergehenden Anspruch und Anspruch 3, **dadurch gekennzeichnet, dass** eine gemeinsame Knicklinie (54) für beide zweiten Hohlräume (22) vorgesehen ist, sodass durch ein einzelnes Knicken von außen beide im zweiten Hohlkörper (21) befindliche Ampullen (24) aufgebrochen werden können.

11. Vorrichtung (10) nach einem der vorhergehenden Ansprüche, **dadurch gekennzeichnet, dass** der erste Hohlkörper (11) ein erstes Verbindungselement (61) aufweist und der zweite Hohlkörper (21) ein zweites Verbindungselement (62) aufweist, wobei das erste Verbindungselement (61) und das zweite Verbindungselement (62) so eingerichtet sind, dass der erste Hohlkörper (11) und der zweite Hohlkörper (21) manuell und lösbar miteinander verbindbar sind.

12. Vorrichtung (10) nach dem vorhergehenden Anspruch, **dadurch gekennzeichnet, dass** die Vorrichtung (10) ferner einen Adapter (56) aufweist, welcher mit dem ersten Verbindungselement (61) verbindbar ist und so eingerichtet ist, dass eine Mischung zur Herstellung von Knochenzement (34) im ersten Hohlkörper (11) zurückgehalten wird.

13. Vorrichtung (10) nach einem der zwei vorhergehenden Ansprüche, **dadurch gekennzeichnet, dass** die Vorrichtung (10) ferner ein Austragelement (58) mit einem dritten Verbindungselement (63) aufweist, welches an das erste Verbindungselement (61) des ersten Hohlkörpers (11) angeschlossen werden kann.

14. Vorrichtung (10) nach dem vorhergehenden Anspruch, **dadurch gekennzeichnet, dass** der erste Hohlkörper (11) aus einem Material mit einer Shore A-Härte zwischen 30 und 80 hergestellt ist.
